# EUROPÄISCHE PATENTSCHRIFT

(11) **EP 4 511 648 B1**
(45) Veröffentlichungstag und Bekanntmachung des Hinweises auf die Patenterteilung: **02.09.2026**
(21) Anmeldenummer: 23718972.5
(22) Anmeldetag: 11.04.2023
(51) Int. Cl.: G01N 30/88, G01N 33/22

(54) **VORRICHTUNG ALS SYSTEM VON MODULEN ZUR PROBENNAHME, VORKONZENTRATION UND ANALYSE VON SPRENGSTOFFEN IN WÄSSERIGEN LÖSUNGEN UND/ODER UXO UND MUNITIONSALTLASTEN IM MEER UND AUF DEM MEERESBODEN SOWIE VERFAHREN DAZU**
DEVICE IN THE FORM OF A SYSTEM OF MODULES FOR SAMPLING, PRE-CONCENTRATING AND ANALYZING EXPLOSIVES IN AQUEOUS SOLUTIONS AND/OR UNEXPLODED ORDNANCE AND MUNITIONS DUMPED IN THE SEA AND ON THE SEABED, AND RELATED METHOD
DISPOSITIF UTILISÉ EN TANT QUE SYSTÈME DE MODULES POUR LE PRÉLÈVEMENT D'ÉCHANTILLONS, LA PRÉCONCENTRATION ET L'ANALYSE DE MATIÈRES EXPLOSIVES DANS DES SOLUTIONS AQUEUSES ET/OU DES SITES POLLUÉS PAR DES MUNITIONS EXPLOSIVES EXPLOSÉES ET NON EXPLOSÉES EN MER ET SUR LE FOND MARIN, ET PROCÉDÉ CORRESPONDANT

(30) Priorität: 22.04.2022 DE 102022109819; 22.04.2022 DE 202022102178 U
(43) Veröffentlichungstag der Anmeldung: 26.02.2025
(73) Patentinhaber: Geomar Helmholtz-Zentrum für Ozeanforschung Kiel, 24148 Kiel (DE)
(72) Erfinder: BECK, Aaron, 24116 Kiel (DE); ESPOSITO, Mario, 24106 Kiel (DE); SCHWENK, Arne, 24113 Kiel (DE)
(74) Vertreter: patcare Patentanwälte Partnerschaft mbB
(86) Internationale Anmeldenummer: PCT/DE2023/100266
(87) Internationale Veröffentlichungsnummer: WO 2023/202744

(56) Entgegenhaltungen:
- WO-A1-2019/237135
- US-A- 4 252 537
- US-A1- 2004 235 187
- US-A1- 2016 011 154

## Beschreibung

Die Erfindung betrifft eine Vorrichtung als System von Modulen zur Probennahme, Vorkonzentration und Analyse von Sprengstoffen in wässerigen Lösungen, insbesondere von UXO und Munitionsaltlasten im Meer und auf dem Meeresboden, und ein darauf gerichtetes Verfahren.

Unter Sprengstoffen auch Explosivmitteln werden chemische Verbindung oder eine Mischung chemischer Verbindungen verstanden, die unter bestimmten Bedingungen sehr schnell reagieren und dabei eine große Energiemenge freisetzen kann.

Unter einer Vorrichtung als System ist im Sinne der Erfindung ein aus mehreren technischen Teilen in Modulen geordnetes zusammengesetztes Ganzes zu verstehen, das aus verschiedenen Komponenten mit unterschiedlichen technischen Eigenschaften besteht, die aufgrund bestimmter geordneter Beziehungen untereinander als gemeinsames Ganzes betrachtet werden.

Die erfinderische Vorrichtung als System von Modulen zur Probennahme, Vorkonzentration und Analyse von Sprengstoffen umfasst ein ex situ Modul zur Probennahme und Vorkonzentration. Ein externes Modul zur in situ Probennahme und Vorkonzentration ist mit dem ex situ Modul verbindbar. Die erfinderische Vorrichtung als System ist damit auf alle Arten der Probennahme, Vorkonzentration und Analyse von Sprengstoffen in wässerigen Lösungen flexibel ausgerichtet.

Das Verfahren zeigt Verfahrensschritte zur Probennahme, -analyse und -behandlung durch die erfinderische Vorrichtung als System von Modulen zur Probennahme, Vorkonzentration und Analyse von Sprengstoffen auf.

Um die Erfindung zu verdeutlichen sind die verwendeten Begriffe und Akronyme bei der vorliegenden Erfindung sinngemäß wie folgt definiert:
UXO In den verschiedensten Regionen der Weltmeere wurden bei und nach kriegerischen Auseinandersetzungen Kriegsgeräte versenkt, die als sogenannte Blindgänger oder **Unexploded Ordnance** oder **UXO** bezeichnet werden.

**TNT** ist eine Sprengstoffverbindung, die allgemein **Trinitrotoluol** oder TNT genannt wird. Die Verbindung entsteht durch Nitrierung von Toluol mittels Nitriersäure, einer Mischung von Salpeter- und Schwefelsäure. TNT wurde erstmals im Jahre 1863 von Julius

Wilbrand (1839-1906) synthetisiert. Seit 1901 wird TNT im großen Stil produziert und eingesetzt. Das sogenannte TNT-Äquivalent dient als Maßstab für die bei einer Explosion freiwerdende Energie.

**RDX** ist eine Sprengstoffverbindung, die allgemein Hexogen, Cyclotrimethylentrinitramin, Cyclonit, T4 oder **Research Department Explosive** bzw. **Royal Demolition Explosive,** kurz **RDX)** genannt wird. RDX ist ein hochbrisanter, giftiger Sprengstoff aus der Gruppe der Nitramine, der während des Zweiten Weltkriegs in großen Mengen hergestellt wurde und immer noch eingesetzt wird. RDX wurde 1898 von dem Berliner Chemiker und pharmazeutischen Unternehmer Georg Friedrich Henning als Explosivstoff zur technischen Verwertung patentiert (DE 104 280 A).

ADNT ist zusammen die Verbindungen 2-Amino-4,6-dinitrotoluol und 4-Amino-2,6-dinitrotoluol. Die sind primäre Reduktions- und mikrobielle Abbauprodukte des militärischen Sprengstoffs 2,4,6-Trinitrotoluol (TNT). ADNT bildet sich relativ schnell, wenn TNT sich freilöst, und ist in der Lage, in der Umwelt zu persistieren.

**DNB** ist eine Sprengstoffverbindung, die allgemein **Dinitrobenzol** oder DNB genannt wird und als Grundsubstanz für Ersatzsprengstoffe eingestuft wird.

**ESI-MS** ist eine Vorrichtung zur Analyse mit Hilfe der **Elektrospray-Ionisations-**Massenspektrometrie oder **ESI-MS** oder **ESMS.** ESI-MS ist eine seit 1968 bekannte Technik zur Erzeugung von Ionen, die in der Massenspektrometrie verwendet wird. Die Technik hat den Vorteil, dass Ionen unter Atmosphärendruck erzeugt werden und ist ein bevorzugtes Ionisationsverfahren zur Analyse von Biomolekülen. Das ESI-MS wird zur Bestimmung von Molekülmassen, zur Analyse und Sequenzierung von Proteinen und Oligonukleotiden und quantitative Bestimmung von Arzneimitteln, Pestiziden und Sprengstoffen auch bei relativ kleinen Molekülen angewendet.

**UV-S** ist eine Vorrichtung zur Analyse mit Hilfe der **UV-Spektrophotometrie** oder **UV-S.** UV-S ist ein spektroskopisches Verfahren der optischen Molekülspektroskopie, das elektromagnetische Wellen des ultravioletten (UV) Lichts nutzt.

**HPLC** ist eine Vorrichtung zur Anwendung in der **Hochdruckflüssig-chromatographie** oder **HPLC. HPLC-Pumpen** haben die Funktion einen Ionenaustausch in einer Probe mit konstantem Fluss gegen einen hohen Druck zu fördern. Dabei sollen sie pulsationsarm und totvolumenarm arbeiten, eine hohe Langzeitkonstanz und Zuverlässigkeit zeigen, damit qualitative und quantitative Aussagen aus den Detektorsignalen möglich werden.

**ACN** ist ein organisches labortechnisches Lösungsmittel. **Acetonitril** oder **ACN** wird auch als Lösungsmittel in der NMR-Spektroskopie in Verbindung mit Massenspektrometrie, IR-Spektroskopie und UV/VIS-Spektroskopie genutzt.

Die Erschließung von Offshore-Ressourcen und der Umweltschutz der Meere werden durch das Vorhandensein von UXO und Munitionsaltlasten im Meer und auf dem Meeresboden erschwert. Neben dem Explosions- und Sicherheitsrisiko enthält diese Munition zytotoxische, genotoxische und krebserregende Chemikalien, die mit konventionellen Sprengstoffen, chemischen Kampfstoffen und Munitionsbestandteile verbunden sind.

Die Räumung von Unterwassermunition ist aufgrund der Gefahren, die die mit der versehentlichen Detonation und dem Austritt giftiger Chemikalien verbunden sind sehr gefährlich.

Aus dem Stand der Technik sind eine Reihe von Sprengstoff-Analysesystemen z.B. für den Einsatz zur Gepäckkontrolle an Flughäfen oder in der Zugangskontrolle bekannt, die jedoch für den Offshore Einsatz ungeeignet sind.

Die Druckschrift US 2004 / 0 101 900 A1 zeigt ein Assay zum Nachweis und zur Quantifizierung von TNT und mit TNT verwandten Verbindungen in wässrigen Lösungen, einschließlich Meerwasser, enthaltend ein TNT-Analogon, das an ein TNT-Erkennungselement gebunden ist, wobei eine messbare Eigenschaftsänderung auftritt, wie z.B. eine Abnahme der Fluoreszenzemissionsintensität des TNT-Analogons, wenn eine Testprobe, die TNT oder verwandte Verbindungen enthält, zu dem Assay hinzugefügt wird, was dazu führt, dass freies TNT das TNT-Analogon von dem TNT-Erkennungselement verdrängt. Ebenfalls offengelegt wird das zugehörige Verfahren zum Nachweis und zur Quantifizierung von TNT und mit TNT verwandten Verbindungen.

Die Druckschrift US 2016 / 0 011 154 A1 offenbart HPLC-Methoden zum Nachweis, zur Identifizierung und zur Quantifizierung von Munitionsverbindungen oder Munitionsmaterialien werden offengelegt. Unempfindliche Munitionssprengstoffe (IMX) können zusammen mit herkömmlichen Munitionsverbindungen wie 2,4,6-Trinitrotolen (TNT) in einer einzigen Säulenanalyse nachgewiesen werden. Die Verfahren eignen sich auch für die analytische Auswertung von Bodenproben, wässrigen Proben wie Grundwasserproben und Gewebeproben, die unempfindliche Munitionssprengstoffe (IMX) enthalten.

Weiter wird in der Veröffentlichung RUSSELL, A. L. [et al.]: Analysis of munitions constituents in IMX formulations by HPLC and HPLC-MS. In: Talanta, Vol. 128, 2014, S. 524-530 ausgeführt, dass der Einsatz empfindlicher Munitionssprengstoffe (IMX) zunimmt, da die Armee versucht, bestimmte herkömmliche Munitionsbestandteile wie 2,4,6-Trinitrotolen (TNT) zu ersetzen, um die Sicherheit zu erhöhen. Die IMX-Formulierungen sind stabiler und daher weniger anfällig für eine versehentliche Detonation, während sie gleichzeitig so konzipiert sind, dass sie die Leistung der bisherigen Materialien erreichen. Zwei Formulierungen, IMX 101 und 104, werden als Ersatz für TNT in Artilleriegeschossen bzw. Mörsern der Zusammensetzung B der Armee untersucht. Die chemischen Formulierungen von IMX-101 und 104 bestehen aus vier Bestandteilen: 2,4-Dinitroanisol (DNAN), 3-Nitro-1,2,4-triazol-5-on (NTO), 1-Nitroguanidin (NQ) und Hexahydro-1,3,5-trinitro-1,3,5-triazin (RDX), die in verschiedenen Verhältnissen gemischt werden, um die gewünschte Wirkung zu erzielen. In der vorliegenden Arbeit wird die Analyse der Bestandteile mittels Einsäulen-HPLC-UV-ESI-MS beschrieben. Die ermittelten Nachweisgrenzen stimmen mit ähnlichen HPLC-Analysen von Verbindungen überein und liegen zwischen 7 und 9 µg/L. Es werden Gradienten-Mobilphasen verwendet, um die Trennung der 4 Zielverbindungen in komplexeren Gemischen anderer Begleitverbindungen zu ermöglichen. Massenspektren werden verwendet, um die Identität des Analyten mit der chromatographischen Retentionszeit zu bestätigen.

In der Druckschrift US 2004/0235187 A1 wird ein Gerät zur Probenahme und Analyse von Analyten in einer flüssigen Probe offenbart. Eine flüssige Probe wird mit Hilfe einer chromatographischen Säule vorkonzentriert, bevor sie in einem zweistufigen Analysegerät analysiert wird. Ein Hochleistungs-Flüssigkeitschromatographiesystem mit Ultraviolett-Detektion kann in Verbindung mit einer elektrochemischen Detektion für die Analyse von Umweltkontaminanten, einschließlich Sprengstoffrückständen, verwendet werden. Die vorliegende Erfindung ermöglicht auch die Vor-Ort-Analyse solcher Verunreinigungen. Die vorliegende Erfindung sieht auch Verfahren zur Analyse der Bestandteile einer Flüssigkeitsprobe vor, einschließlich Verfahren zur Vor-Ort-Analyse.

Die derzeit verfügbaren und ausgereiften geophysikalischen Technologien zur zerstörungsfreien Messung von physikalischen bzw. chemischen Anomalien sind derzeit nicht in der Lage, Munition auf dem Meeresboden universell zu identifizieren.

Die vorliegende Erfindung beseitigt die Mängel des Standes der Technik.

Der vorliegenden Erfindung liegt die Aufgabe zugrunde mit der Probennahme eine direkte Erkennung von explosivem Material mit einer eindeutigen Signatur für Objekte, die geräumt werden müssen zu liefern, um die Freisetzung und chemischer Kontamination im Meer durch Munition zu verhindern.

Eine weitere Aufgabe der Erfindung ist es eine nahezu Echtzeit-Detektionstechnologie für chemische Stoffe, auch in situ bereitzustellen.

Eine weitere Aufgabe ist es mehrere chemische Verbindungen gleichzeitig zu analysieren und einen spezifischen Nachweis von gelösten Sprengstoffen und chemischen Kampfstoffen im Meerwasser zu detektieren.

Eine weitere Aufgabe ist es ein seegängiges Gerät zum Nachweis von Chemikalien,
die mit nicht zur Wirkung gelangten Kampfmitteln in der Meeresumwelt in Verbindung stehen, bereit zu stellen.

Die erfinderische Vorrichtung als System von Modulen zur Probennahme, Vorkonzentration und Analyse von Sprengstoffen soll als Gerät für eine unterstützende Munitionsdetektionstechnologie in Kombination mit bestehenden ausgereiften geophysikalischen Technologien zum Einsatz kommen, um in Kombination mit bekannten Sensoren eine Entdeckungswahrscheinlichkeit und bessere Informationen zur Charakterisierung von Sprengstoffen und chemischen Kampfstoffen im Meerwasser bereit zu stellen.

Die gestellte Aufgabe wird im unveröffentlichten EU-Projekt ExPloTect (ExPloTect: "Ex-situ, near-real-time exPlosive compound deTection in seawater") des Europäischer Meeres- und Fischereifonds, Projektnummer: 863693, https://www.explotect.eu wie folgt erläutert: Das Projekt **ExPloTect: "Ex-situ-Nachweis von explosiven Verbindungen in Meerwasser nahezu in Echtzeit, beschäftigt sich mit Problemen, der** Erschließung von Offshore-Ressourcen und dem damit verbundenen Umweltschutz bei Vorhandensein von UXO und Reliktmunition auf dem Meeresboden. Zusätzlich zu den Explosions- und Sicherheitsrisiken enthält diese Munition zytotoxische, genotoxische und karzinogene Chemikalien, die mit herkömmlichen Sprengstoffen, chemischen Kampfstoffen und Munitionsstrukturkomponenten in Verbindung gebracht werden. Aufgrund der Gefahren, die mit einer versehentlichen Detonation und dem Austreten giftiger Chemikalien verbunden sind, besteht ein kritischer Bedarf, Unterwassermunition zu räumen."

**Gelöst** wird diese Aufgabe mit der erfinderischen Vorrichtung als System von Modulen zur Probennahme, Vorkonzentration und Analyse von Sprengstoffen sowie einem Verfahren zur Probennahme, Probenanalyse und Probenbehandlung.

Die Vorrichtung ist als System von Modulen zur Probennahme, Vorkonzentration und Analyse von Sprengstoffen in wässerigen Lösungen und/oder UXO und Munitionsaltlasten im Meer und auf dem Meeresboden ausgestaltet,
wobei
die Vorrichtung als System wenigstens aufweist ein Gehäuse in dem zumindest ein erstes Flüssigkeitssystem Modul und/oder ein zweites Flüssigkeitssystem Modul und zumindest ein Analysesystemmodul vorgesehen sind
mit folgenden Bauteilen im ersten Flüssigkeitssystem Modul:
   - zumindest drei HPLC-Pumpen, mit unterschiedlichen Pumpkapazitäten;
   - zumindest zwei Ventile;
   - zumindest einem Probenwahlventil, als ein 6-fach Ventil mit steuerbaren Einstellwahlmöglichkeiten für Abfall- und Injektionspositionen;
   - zumindest einem Inline-Filter;
mit folgenden Bauteilen im zweiten Flüssigkeitssystem Modul:
   - zumindest einer HPLC-Pumpe;
   - zumindest einer elektronischen Steuerplatine;
und mit folgenden Bauteilen in dem Analysesystemmodul:
   - zumindest einem Mischer;
   - zumindest einem Ventil, als Umleitungsventil;
   - zumindest einer HPLC-Säulenheizung;
   - zumindest einem UV-System mit Kapillarwellenleiter und Spektralphotometer;
dadurch gekennzeichnet, dass
   - das erste Flüssigkeitssystem Modul
      - zumindest eine Spritzenpumpe mit 4-Port-Verteilerventil aufweist und
      - die zumindest zwei Ventile über steuerbare Transfer- und Lade-/Einspritzpositionen verfügen;
   - das Analysesystemmodul über zumindest ein ESI-MS verfügt;
   - das zweite Flüssigkeitssystem Modul
      - ein Druckgehäuse;
      - eine erste Druckgehäuse-Endkappe A;
      - eine zweite Druckgehäuse-Endkappe B;
      - zumindest einen HPLC-Säulenselektor, mit zumindest zwei HPLC Säulen,
      als Festphasenextraktionssäulen für die Probennahme aufweist;
   - bei Vorliegen eines zweiten Flüssigkeitssystem Moduls zumindest eine der Druckgehäuse-Endkappen Verbindungselemente aufweist, die das zweite Flüssigkeitssystem Modul mit einem jeweiligen Analysesystem Modul lösbar
   verbindet.

Verfahrensgemäß ist das Verfahren zur Probennahme, Probenanalyse und Probenbehandlung mit einer erfindungsgemäßen Vorrichtung als System mit zumindest folgenden Schritten ausgebildet:
a. Vorreinigung (100)
b. Beladen der Probe (101)
c. Überführung (102)
d. finale Probenvorbereitung (103)
e. chromatographische Trennung (104)
f. Massenspektrometrie (MS)-Detektion (105)
g. Spektralphotometrischer Nachweis im ultravioletten Bereich (UV) (106)
h. Nachreinigung (107).

Weiter kann eine jeweilige nächste Folge-Probe oder Diskrete-Probe einer Probennahme mit Erreichen einer ersten Probe im Schritt c. Überführung, gleichzeitig auf die erste SPE-Säule (9) geladen werden.

Die Vorrichtung als System von Modulen zur Probennahme, Vorkonzentration und Analyse von Sprengstoffen in wässerigen Lösungen, insbesondere von UXO und Munitionsaltlasten im Meer und auf dem Meeresboden, und ein darauf gerichtetes Verfahren führt eine zeitlich direkte Probennahme (ex situ und/oder in situ), Probenkonzentration und Analyse von Sprengstoffverbindungen (TNT, RDX, ADNT, DNB) aus Meerwasser durch. Dabei werden die gesuchten Zielverbindungen durch HPLC getrennt und durch Elektrospray-Ionisations-Massenspektrometrie (ESI-MS) und UV-Spektrophotometrie im Analyse Modul nachgewiesen.

Das erste Flüssigkeitssystem Modul reichert dabei großvolumige Meerwasserproben (bis zu mindestens 1 L) an und verwendet ein Elutions-Verdünnungs-Zweisäulenverfahren, um einen 5000-fachen Anreicherungsfaktor zu erreichen. Umschaltventile und Probenschleifen werden dabei eingesetzt, um die Säulenspülzeiten kurz zu halten und störende Verbindungen zu reduzieren. Eine Spritzenpumpe wird zur Injektion von Standardlösungen für die Kalibrierung verwendet.

Die vorkonzentrierte Probe wird mittels HPLC getrennt, wobei ein Umschaltventil unerwünschte organische Verbindungen eliminiert. Der Probenstrom wird dann zwischen dem ESI-MS- und dem UV-Detektor zur doppelten Detektion im Analyse Modul aufgeteilt. Das System ist von der Probenentnahme bis zur Datenanalyse vollständig automatisiert und benötigt derzeit weniger als 15 Minuten pro Probe.

Das zweite Flüssigkeitssystem Modul arbeitet im Unterschied zum ersten Flüssigkeitssystem Modul mit Probennahme, Vorkonzentration in situ und ist damit ein eigenständiges Unterwassergerät zur Probennahme und Vorkonzentration von gelösten Sprengstoffen im Meerwasser direkt an der UXO und Reliktmunition.

Das zweite Flüssigkeitssystem Modul führt im Wesentlichen den Schritt 101 des ersten Flüssigkeitssystem Moduls aus, kann aber während eines einzigen Einsatzes bis zu zehn Proben sammeln. Er besteht aus einer HPLC-Pumpe und einem HPLC-Säulenselektor, die in einem wasserdichten Druckgehäuse untergebracht sind. Die Proben werden gefiltert und nacheinander durch Pumpen durch eine Reihe von zehn Festphasenextraktionssäulen (SPE) vorkonzentriert. Die Pumpe und der Säulenselektor werden über eine elektronische Platine durch Ein- und Ausschalten der Stromversorgung gesteuert. Das zweite Flüssigkeitssystem Modul wird direkt an das Analyse Modul angeschlossen und die gesammelten Proben werden dann durch das Analyse Modul analysiert und mit der zugehörigen Software datentechnisch umgesetzt und auf einem Display dargestellt.

Nachfolgend wird die Erfindung anhand der beiliegenden Abbildungen in der **Abbildungsbeschreibung** beschrieben, wobei diese die Erfindung erläutern sollen und nicht beschränkend zu werten sind:
Es zeigen:
- Abb. 1: eine beispielhafte Vorrichtung als System in Schnittdarstellung mit den Bauteilen der Module erstes Flüssigkeitssystem Modul 4 und Analysesystem Modul 22 ohne das zweite Flüssigkeitssystem Modul 30 (vgl. dazu auch Abb. 4);
- Abb. 2: beispielhafte Screenshots aus einer Datenanzeige auf dem externen Rechner mit Display 21 einer Auswertesoftware (systemeigene Software) für eine Meerwasserprobe, einer Messung mit dem Analysesystem Modul 22 nach Abb. 1;
- Abb. 3: beispielhafte Verfahrensschritte für den Ablauf einer Messung mit einer Vorrichtung als System 1;
- Abb. 4: ein beispielhaftes zweites Flüssigkeitssystem Modul 30. Der Aufbau des Moduls 30 ist im Längsschnitt (Abbildung oben) und in Aufsicht auf die Druckgehäuse-Endkappe A und Druckgehäuse-Endkappe B gezeigt und
- Abb. 5: eine beispielhafte Vorrichtung als System 1 in perspektivischer Darstellung mit den Modulen des Systems, dem ersten Flüssigkeitssystem Modul 4, dem Analysesystem Modul 22 und dem zweiten Flüssigkeitssystem Modul 30. Ergänzend sind der Vollständigkeit halber die externen Komponenten externes Luftkompressor/Stickstoffgenerator-Modul 2, externer Rechner mit Display 21 und ein externer Abfall-Kanister 28 gezeigt.

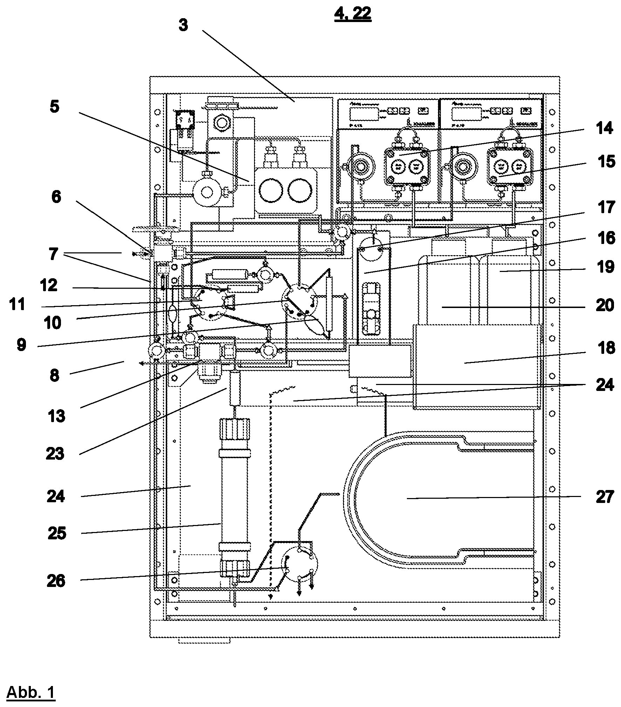

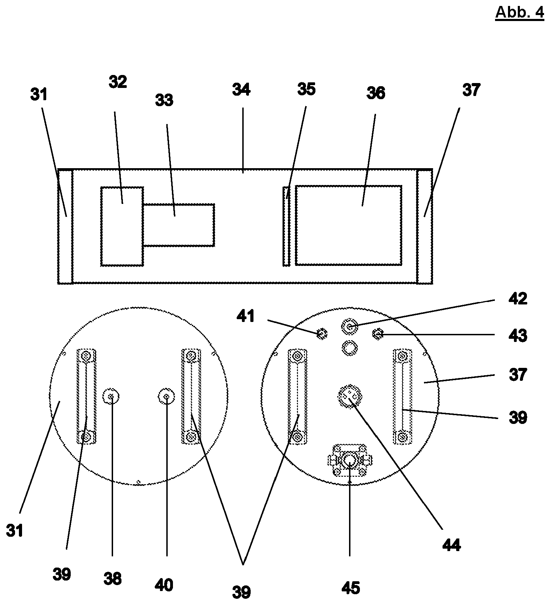

**Abb. 1** zeigt eine beispielhafte Vorrichtung als System 1 in Schnittdarstellung mit den Bauteilen der Module erstes Flüssigkeitssystem Modul 4 und Analysesystem Modul 22. Das zweite Flüssigkeitssystem Modul 30 der Vorrichtung als System 1 ist aus Gründen der Übersichtlichkeit in Abb. 4 gezeigt und weiter unten erläutert.

Die beispielhafte Vorrichtung als System 1 zeigt die Module 4 und 22, in Schnittdarstellung mit den Bauteilen eines ersten Flüssigkeitssystem Moduls 4 und eines Analysesystem Moduls 22 in einem Gehäuse 3. Die Module 4, 22 sind für den Einbau in ein standardmäßiges 19"-Elektronik-Rack Gehäuse 3 geeignet, können jedoch auch an andere bautechnische Erfordernisse angepasst werden. Für den Einsatz vor Ort oder auf Schiffen wird das 19"-Elektronik-Rack Gehäuse bevorzugt in einem tragbaren, robusten 19"-Einschubgehäuse installiert. Die erste HPLC-Pumpe 5 besitzt eine hohe Pumpkapazität, z.B. 500 ml/min maximale Kapazität mit einem Pumpenkopf aus Titan und einer Steuerung über eine systemeigene Software. Die zweite HPLC-Pumpe 14 und die dritte HPLC-Pumpe 15 besitzen eine geringere Pumpkapazität, z.B. eine jeweilige maximale Kapazität von 10 ml/min und eine Steuerung über die systemeigene Software. Das erste Ventil 10 und das zweite Ventil 11 verfügen jeweils über Transfer- und Lade-/Einspritzpositionen mit Steuerung über die systemeigene Software. Das Probenwahlventil 6 ist ein 6-fach Ventil mit Einstellwahlmöglichkeiten für Abfall- und Injektionspositionen und eine Steuerung über die systemeigene Software. Über das Probenwahlventil 6 wird eine Wahl zwischen Online-Probenströmen oder diskreten Probenströmen getroffen. Die Einstellung wird zu Beginn einer Beprobung manuell gesteuert und wird dann bis zu einer erneuten Steuerung, z.B. für die Messung einer diskreten Probe, fortgesetzt bzw. nach dem gesamten Durchlauf einer Online- Probenserie beendet. Online-Proben werden z.B. aus dem gepumpten Seewasser des Schiffes (Reinseewasser oder Motorkühlwasser) oder einer über die Bordwand geführten Pumpe entnommen. Der Inline-Filter 13 besteht bevorzugt aus einem gesinterter Edelstahlfilter mit 5 µm Porengröße. Die Spritzenpumpe 16 mit 4-Port-Verteilerventil 17 verfügt bevorzugt über eine 1-mL-Spritze mit einer Steuerung über die systemeigene Software. Die ultrakompakte HPLC-Säulenheizung 25 hält die HPLC-Säule auf der eingestellten Temperatur konstant, bevorzugt auf 40±0,05°C. Das jeweilige UV-System 24 besitzt bevorzugt eine Xenon-Lichtquelle mit 1-m-Kapillarwellenleiter und Spektralphotometer, wie beispielsweise eine PX-2 Pulsed Xenon Lamp und ein FLAME-S-UV-VIS Spektrometer (Fa. Ocean Insight). Als kompaktes ESI-MS Gerät wird ein bekanntes Produkt wie z.B. ein Microsaic MiD^{®} 4500 der Firma Microsaic Systems verbaut.

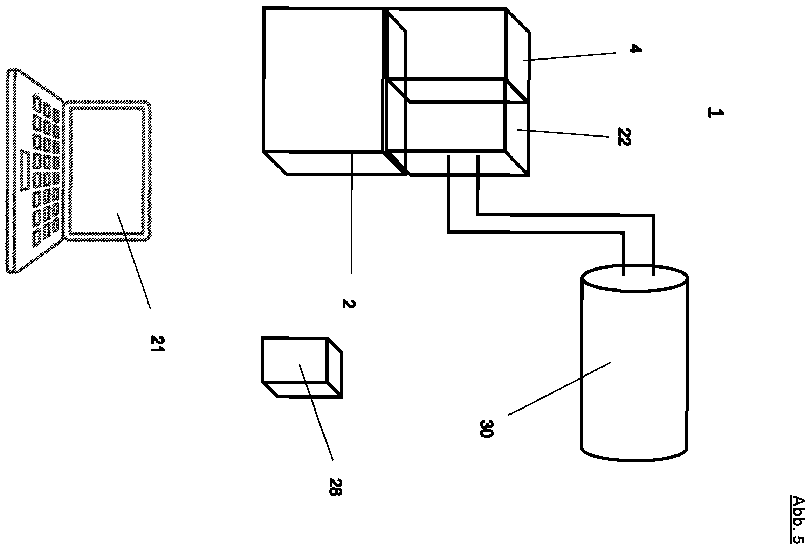

Die beispielhafte Vorrichtung als System 1 wird bei Bedarf mit hochreinem, komprimiertem Stickstoff aus einem externen Luftkompressor / Stickstoffgenerator-Modul 2 versorgt, das in einem separaten tragbaren, robusten, bevorzugt als 19"-Rack ausgeführten Gehäuse untergebracht ist, wie in **Abb. 5** schematisch gezeigt.

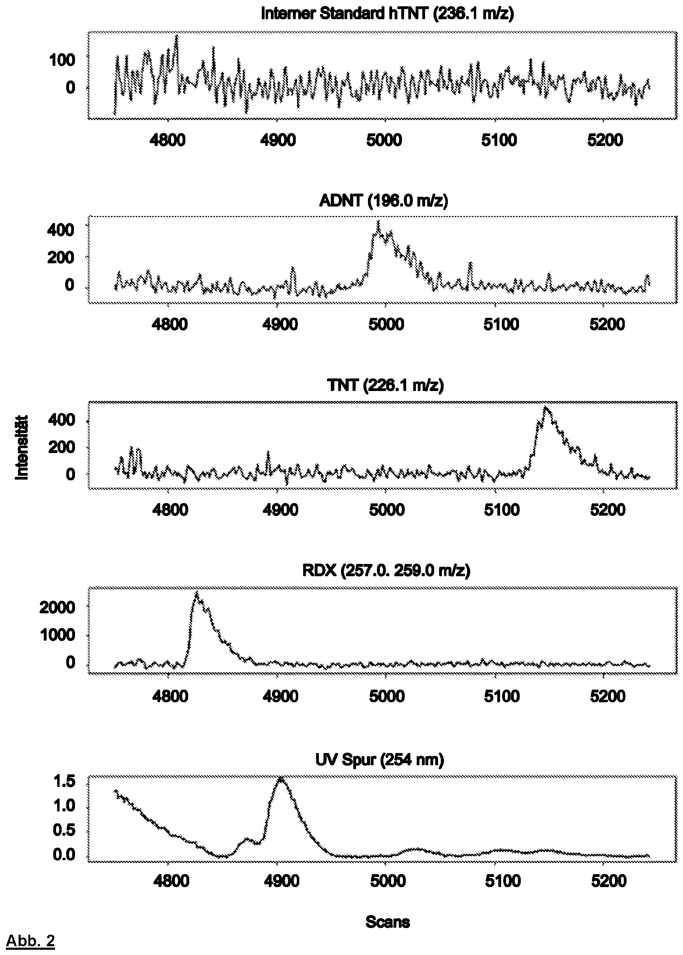

Die **Abb. 2** zeigt beispielhaft Screenshots aus einer Datenanzeige einer Messung auf einem externen Rechner mit Display 21 mit der erfinderischen Vorrichtung als System 1 von Modulen zur Vorkonzentration und Analyse (4 bzw. 30, 22) von Sprengstoffen durch eine Auswertesoftware (systemeigene Software) für eine Meerwasserprobe, der 25 ng einer explosiven Zielverbindungen hinzugefügt wurden.

Von oben nach unten zeigen: die erste Kurve eine Messkurve einer "neutralen" Probe mit **ESI-MS** von masse 236.1 amu (Stabil-Isotopen-labillerte TNT), als interner Standard, bei eine Probe der kein interner Standard hinzugefügt wurde; die zweite Kurve eine Messung einer Probe mit **ESI-MS** mit einem Peak von **ADNT;** die dritte Kurve eine Messung einer Probe mit **ESI-MS** mit einem Peak von **TNT;** die vierte Kurve eine Messung einer Probe mit **ESI-MS** mit einem Peak von **RDX;** und die fünfte Kurve eine Messung einer Probe mit **UV-S** mit einem Peak von **DNB.** Die niedrigen Peaks in der UV-S Kurve sind unbekannte natürliche organische Stoffe. Die jeweiligen Peaks sind durch einen Pfeil gekennzeichnet. Die Kurven zeigen auch relativ zueinander eine gute Peaktrennung auf.

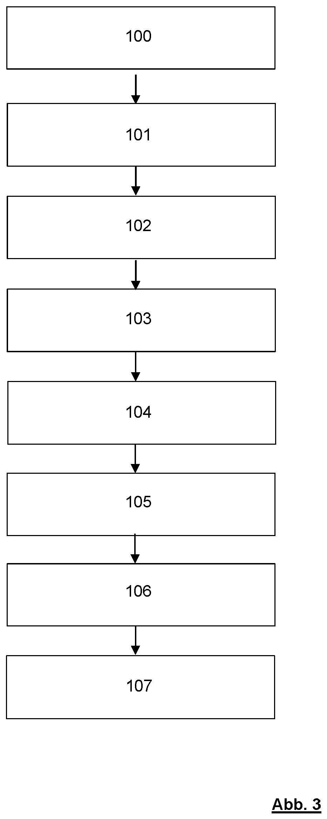

**Abb. 3** zeigt beispielhaft die Verfahrensschritte einer Messung mit der erfinderischen Vorrichtung als System 1 von Modulen zur Vorkonzentration und Analyse (4 bzw. 30, 22) von Sprengstoffen (nach **Abb.1** **bzw. 4).**
1. Schritt: Vorreinigung 100: Die Ventile 10 und 11 befinden sich in der Übergabestellung. Das dritte Ventil (Umleitungsventil) 26 wird in eine Abfallposition gebracht. Die Festphasenextraktionssäulen 9 und 12 werden durch die dritte HPLC-Pumpe 15 mit 80 % Acetonitril (ACN) gereinigt. Das verbrauchte ACN wird über den Inline-Filter 13 zurück gespült und wird dann über das dritte Ventil (Umleitungsventil) 26 in die Sammlung gefährlicher Abfälle in den externen Abfall-Kannister 28 umgeleitet.
2. Schritt: Beladen der Probe 101: Die Ventile 10 und 11 werden in die Position Laden/Einspritzen gebracht. Das Probenwahlventil 6 wird entweder auf Online- oder diskreten Probenstrom eingestellt. Die Probe wird über die erste HPLC-Pumpe 5 mit Meerwasser gepumpt. Gleichzeitig werden dem Probenstrom über die Spritzenpumpe 16 und ihr integriertes 4-Port-Verteilerventil 17 entweder interne oder externe Standards zugesetzt. Partikel in der Probe werden durch den Inline-Filter 13 entfernt. Die Probe durchläuft die Proben-SPE-Säule, erste Festphasenextraktionssäule 9. Die explosiven Zielverbindungen werden auf der Proben-SPE-Säule, erste Festphasenextraktionssäule 9 zurückgehalten. Das abfließende Meerwasser wird zurück ins Meer geleitet, z.B. über die Bordwand oder durch ein Abflussrohr.
3. Schritt: Überführung 102: Die Ventile 10 und 11 werden in eine Transferstellung gebracht. Das dritte Ventil (Umleitungsventil) 26 wird in die Abfallstellung gebracht. Die analytische Säule, erste Festphasenextraktionssäule 9 wird über die zweite HPLC-Pumpe 14 mit ultrareinem Wasser aus dem Reagenz Behälter Reinstwasser 20 gespült. Dann wird ACN aus dem Reagenz Behälter ACN 19 über die dritte HPLC-Pumpe 15 gepumpt, um die Zielverbindungen von der Proben-SPE-Säule, erste Festphasenextraktionssäule 9 zu eluieren. Der ACN-Strom mit den Zielverbindungen wird online mit Reinstwasser verdünnt, und die Verbindungen werden auf der analytischen Säule, zweite Festphasenextraktionssäule 12 zurückgehalten. Mit dem Abwasser werden Partikel aus dem Inline-Filter 13 zurückgespült und über das dritte Ventil (Umleitungsventil) 26 in die Sondermüllsammlung, z.B. einen externen Abfall-Kanister (28) abgeleitet.

Die nächste Folge-Probe oder Diskrete-Probe wird in dieser Phase, d. h. im 3. Schritt Überführung 102, gleichzeitig auf die erste SPE-Säule 9 geladen.
4. Schritt: Endgültige Probenvorbereitung 103: Die Ventile 10 und 11 werden auf die Position Laden/Einspritzen eingestellt. Das dritte Ventil (Umleitungsventil) 26 wird auf die Abfallposition gestellt. Die Zielverbindungen werden von der analytischen Säule, zweite Festphasenextraktionssäule 12 mit über die dritte HPLC-Pumpe 15 gepumptem ACN eluiert. Der ACN-Strom wird online mit über die zweite HPLC-Pumpe 14 gepumptem Reinstwasser und den Mischer 23 verdünnt, um eine ACN-Konzentration (36 %) zu erreichen, die für die chromatografische Trennung und die Analyse mit dem Massenspektrometer ESI-MS 27 erforderlich ist.
5. Schritt: Chromatographische Trennung 104: Die Zielverbindungen in 36%iger ACN werden durch eine C8-HPLC-Säule gepumpt, die in einer ultrakompakten Säulenheizung 25 auf 40±0,05°C gehalten wird. Organische Nicht-Zielverbindungen, die zuerst von der Chromatographiesäule eluieren, werden über das dritte Ventil (Umleitungsventil) 26 in die Sondermüllsammlung, z.B. einen externen Abfall-Kanister (28) abgeleitet. Das dritte Ventil (Umleitungsventil) 26 wird dann wieder umgeschaltet und der HPLC-Säulenausfluss mit den Zielverbindungen wird zum Massenspektrometereinlass in den ESI-MS 27 umgeleitet. Die Verbindungen werden innerhalb von 10 Minuten in der folgenden Reihenfolge getrennt: RDX, DNB, ADNT, TNT.
6. Schritt: Massenspektrometrie (MS)-Detektion 105: Für die MS-Detektion wird im Beispiel ein kompaktes Microsaic MiD^{®} 4500 Elektrospray-Ionisations-Massenspektrometer als ESI-MS 27 verwendet. Der Probenzufluss wird geteilt, wobei 1 µl/min in den Detektor gelangen und die restlichen 999 µl/min zum Auslass abgeleitet werden. Nur RDX, ADNT und TNT sind für den MS-Nachweis geeignet, da DNB schlecht ionisiert ist. Die Zielverbindungen werden im Einzelionenmodus (SIM) bei folgenden Massen TNT: 226,1 m/z; RDX: 257,0 m/z, 259,0 m/z; ADNT: 196,0 m/z analysiert.
7. Schritt: Spektralphotometrischer Nachweis im ultravioletten Bereich (UV) 106: Der Probenauslass des ESI-MS 27 wird in eine 100-cm-Wellenleiterzelle mit einer Xenon-Lichtquelle geleitet. Die Absorption bei 254 nm wird mit einem Spektrophotometer gemessen. Der UV-Nachweis ist in erster Linie für DNB erforderlich, kann aber auch für die anderen Zielverbindungen verwendet werden.
8. Schritt: Nachreinigung 107: Die Ventile 10 und 11 werden in die Lade-/Einspritzposition gebracht. Das dritte Ventil (Umleitungsventil) 26 wird in die Injektionsstellung gebracht. Die Festphasenextraktionssäulen 9 und 12 werden durch die HPLC-Pumpe 15 mit 80 % Acetonitril (ACN) gereinigt.

**Abb. 4** zeigt beispielhaft den Aufbau des zweiten Flüssigkeits-Moduls 30 im Längsschnitt (Abbildung oben) und in Aufsicht auf die Druckgehäuse-Endkappen A und B 31, 37.

Im Längsschnitt des rohrförmigen Druckgehäuses 34 sind ein HPLC-Säulenselektor 32 mit zehn HPLC Säulen 33, Festphasenextraktionssäulen für die Probennahme gezeigt. Weiter sind eine HPLC-Pumpe 36 (Leistung bis zu 500ml/min) und eine elektronische Steuerplatine 35 drucksicher verbaut.

In der Druckgehäuse-Endkappen A 31 sind eine Einlassverbindung zum Analysesystem Modul 38 und ein Wasserauslass/Abfluss zum Analysesystem Modul 40 eingebaut. In der Druckgehäuse-Endkappen B 37 sind ein Pumpenauslass zum T-Filter 41, ein Probenwassereinlass/Pumpeneinlass 42, ein Probeneinlass vom T-Filter zum Säulenselektor 43, eine Strom-/Kommunkationsverbindung, z.B. ein SubConn^{®} Konnektor und ein T-Filter (bevorzugt 5µm) eingebaut.

Im Beispiel sind jeweils zwei Transportgriffe 39 an den Druckgehäuse-Endkappen A und B 31, 37 für eine einfache, sichere Handhabung angebracht

Analog zu dem in Abb. 3 gezeigten Ablauf, erfolgen im zweiten Flüssigkeits- Modul 30 die folgenden Verfahrensschritte:
In Schritt 101: Das Probenwasser wird durch die Druckgehäuse-Endkappe B 37 über den Probenwassereinlass/Pumpeneinlass 42 in die HPLC-Pumpe 36 gepumpt. Die Probe wird aus der Druckgehäuse-Endkappe B 37 durch den Pumpenauslass zum T-Filter 41, durch den T-Filter 45 und über den Probeneinlass vom T-Filter zum Säulenselektor 43 in das Druckgehäuse 34 gepumpt. Die Probe wird über den HPLC-Säulenselektor 32 durch die HPLC-Säulen 33 geleitet. Das abfließende Meerwasser verlässt das Gerät durch den Wasserauslass/Abfluss 40 zum Analysesystem Modul an der Druckgehäuse-Endkappe A 31. Die zehn HPLC-Säulen 33 werden nacheinander ausgewählt, und die Umschaltung des HPLC-Säulenselektor 32 und der Pumpenbetrieb werden durch Ein- und Ausschalten der Stromversorgung über die Strom-/Kommunikationsverbindung 44 und die elektronische Steuerplatine 35 gesteuert.

In Schritt 102: Werden die mit dem zweiten Flüssigkeits Modul 30 gesammelten Proben analysiert, indem die Einlassverbindung zum Analysesystem Modul 38 und der Wasserauslass/Abfluss 40 zum Analysesystem Modul mit dem Analysesystem Modul verbunden werden. Dann wird die Strom-/Kommunikationsverbindung 44 an den Analysesystem Modul angeschlossen und die Proben können über die weiteren Schritte 103 bis 107 analysiert, ausgewertet und dargestellt werden.

**Abb. 5** zeigt beispielhaft den Aufbau einer **Vorrichtung als System 1** des ersten Flüssigkeitssystem Moduls 4 und des Analysesystem Moduls 22 mit dem zweiten Flüssigkeitssystem Modul 30 und den externen Komponenten: Luftkompressor / Stickstoffgenerator Modul 2, Abfall-Kanister 28 und Rechner mit Display 21.

### Bezugszeichenliste

- 1: Vorrichtung als System
- 2: Externes Luftkompressor/Stickstoffgenerator-Modul
- 3: Gehäuse
- 4: erstes Flüssigkeitssystem Modul
- 5: erste HPLC-Pumpe
- 6: Probenwahlventil
- 7: einfließendes Meerwasser
- 8: abfließende Meerwasser
- 9: erste Festphasenextraktionssäule
- 10: erstes Ventil
- 11: zweites Ventil
- 12: zweite Festphasenextraktionssäule
- 13: Inline-Filter
- 14: zweite HPLC-Pumpe
- 15: dritte HPLC-Pumpe
- 16: Spritzenpumpev
- 17: 4-Port-Verteilerventil
- 18: Halterung für Reagenz-Behälter
- 19: Reagenz Behälter ACN
- 20: Reagenz Behälter Reinstwasser
- 21: Externer Rechner mit Display
- 22: Analysesystem Modul
- 23: Mischer
- 24: UV-System
- 25: HPLC-Säulenheizung
- 26: drittes Ventil (Umleitungsventil)
- 27: ESI-MS
- 28: Externer Abfall-Kannister
- 30: zweites Flüssigkeitssystem Modul
- 31: Druckgehäuse-Endkappe A
- 32: HPLC-Säulenselektor
- 33: HPLC-Säulen (10 Festphasenextraktionssäulen)
- 34: Druckgehäuse
- 35: elektronische Steuerplatine
- 36: HPLC-Pumpe (bis zu 500 ml/min)
- 37: Druckgehäuse-Endkappe B
- 38: Einlassverbindung zum Analysesystem Modul
- 39: Transportgriff
- 40: Wasserauslass/Abfluss zum Analysesystem Modul
- 41: Pumpenauslass zum T-Filter
- 42: Probenwassereinlass/Pumpeneinlass
- 43: Probeneinlass vom T-Filter zum Säulenselektor
- 44: Strom-/Kommunikationsverbindung
- 45: T-Filter (bevorzugt 5 µm)
- 100: Vorreinigung
- 101: Beladen der Probe
- 102: Überführung
- 103: finale Probenvorbereitung
- 104: chromatographische Trennung
- 105: Massenspektrometrie (MS)-Detektion
- 106: spektralphotometrischer Nachweis im ultravioletten Bereich (UV)
- 107: Nachreinigung

## Patentansprüche

1. Vorrichtung als System (1) von Modulen (4, 22, 30) zur Probennahme, Vorkonzentration und Analyse von:
- Sprengstoffen in wässerigen Lösungen
und/oder
- UXO und Munitionsaltlasten im Meer und auf dem Meeresboden,
wobei
die Vorrichtung als System (1) wenigstens aufweist ein Gehäuse (3) in dem zumindest ein erstes Flüssigkeitssystem Modul (4) und/oder ein zweites Flüssigkeitssystem Modul (30) und zumindest ein Analysesystemmodul (22) vorgesehen sind mit folgenden Bauteilen im ersten Flüssigkeitssystem Modul (4):
- zumindest drei HPLC-Pumpen (5, 14, 15), mit unterschiedlichen Pumpkapazitäten;
- zumindest zwei Ventile (10, 11);
- zumindest einem Probenwahlventil (6), als ein 6-fach Ventil mit steuerbaren Einstellwahlmöglichkeiten für Abfall- und Injektionspositionen;
- zumindest einem Inline-Filter (13);
mit folgenden Bauteilen im zweiten Flüssigkeitssystem Modul (30):
- zumindest einer HPLC-Pumpe (36) und
- zumindest einer elektronischen Steuerplatine (35)
und mit folgenden Bauteilen in dem Analysesystemmodul (22):
- zumindest einem Mischer (23);
- zumindest einem Ventil (26), als Umleitungsventil;
- zumindest einer HPLC-Säulenheizung (25);
- zumindest einem UV-System (24) mit Kapillarwellenleiter und Spektralphotometer;
**dadurch gekennzeichnet, dass**
- das erste Flüssigkeitssystem Modul (4)
- zumindest eine Spritzenpumpe (16) mit 4-Port-Verteilerventil (17) aufweist und
- die zumindest zwei Ventile (10, 11) über steuerbare Transfer- und Lade-/ Einspritzpositionen verfügen;
- das Analysesystemmodul (22) über zumindest ein ESI-MS (27) verfügt;
- das zweite Flüssigkeitssystem Modul (30)
- ein Druckgehäuse (34);
- eine erste Druckgehäuse-Endkappe A (31);
- eine zweite Druckgehäuse-Endkappe B (37);
- zumindest einen HPLC-Säulenselektor (32), mit zumindest zwei HPLC Säulen (33), als Festphasenextraktionssäulen für die Probennahme aufweist;
- bei Vorliegen eines zweiten Flüssigkeitssystem Moduls (30) zumindest eine der Druckgehäuse-Endkappen (31, 37) Verbindungselemente aufweist, die das zweite Flüssigkeitssystem Modul (30) mit einem jeweiligen Analysesystem Modul (22) lösbar verbindet.

2. Verfahren zur Probennahme, Probenanalyse und Probenbehandlung mit einer Vorrichtung als System (1) nach Anspruch 1 mit zumindest folgenden Schritten:
a. Vorreinigung (100)
b. Beladen der Probe (101)
c. Überführung (102)
d. finale Probenvorbereitung (103)
e. chromatographische Trennung (104)
f. Massenspektrometrie (MS)-Detektion (105)
g. Spektralphotometrischer Nachweis im ultravioletten Bereich (UV) (106)
h. Nachreinigung (107).

3. Verfahren zur Probennahme, Probenanalyse und Probenbehandlung nach dem vorangehenden Anspruch,
**dadurch gekennzeichnet, dass**
eine jeweilige nächste Folge-Probe oder Diskrete-Probe einer Probennahme mit Erreichen einer ersten Probe im Schritt c. Überführung (102), gleichzeitig auf die erste Festphasenextraktionssäule (9) geladen wird.

## Claims

1. A device as a system (1) comprising modules (4, 22, 30) for the sampling, preconcentration and analysis of:
- explosives in aqueous solutions
and/or
- UXO and munitions contamination in the sea and on the seabed,
wherein
the device as a system (1) comprises at least one housing (3) in which at least a first fluid system module (4) and/or a second fluid system module (30) and at least one analysis system module (22) are provided,
with the following components in the first fluid system module (4):
- at least three HPLC pumps (5, 14, 15), with different pumping capacities;
- at least two valves (10, 11);
- at least one sample selection valve (6), in the form of a 6-way valve with controllable setting options for waste and injection positions;
- at least one in-line filter (13);
with the following components in the second fluid system module (30):
- at least one HPLC pump (36) and
- at least one electronic control board (35)
and with the following components in the analysis system module (22):
- at least one mixer (23);
- at least one valve (26), acting as a bypass valve;
- at least one HPLC column heater (25);
- at least one UV system (24) comprising a capillary waveguide and a spectral photometer;
wherein
- the first fluid system module (4) comprises
- at least one syringe pump (16) with a 4-port distribution valve (17) and
- the at least two valves (10, 11) have controllable transfer and loading/injection positions;
- the analysis system module (22) comprises at least one ESI-MS (27);
- the second fluid system module (30) comprises
- a pressure housing (34);
- a first pressure housing end cap A (31);
- a second pressure housing end cap B (37);
- at least one HPLC column selector (32), with at least two HPLC columns (33), as solid-phase extraction columns for sample collection;
- in the presence of a second fluid system module (30), at least one of the pressure housing end caps (31, 37) comprises connecting elements which detachably connect the second fluid system module (30) to a respective analysis system module (22).

2. A method for sample collection, sample analysis and sample treatment using a device as a system (1) according to claim 1, comprising at least the following steps:
a. Pre-purification (100)
b. Loading of the sample (101)
c. Transfer (102)
d. Final sample preparation (103)
e. chromatographic separation (104)
f. mass spectrometry (MS) detection (105)
g. spectrophotometric detection in the ultraviolet (UV) range (106)
h. post-purification (107).

3. Method for sampling, sample analysis and sample treatment according to the preceding claim,
wherein
a respective subsequent sample or discrete sample from a sampling run is loaded onto the first solid-phase extraction column (9) simultaneously upon the arrival of a first sample in step c. Transfer (102).

## Revendications

1. Dispositif en tant que système (1) de modules (4, 22, 30) pour le prélèvement d'échantillons, la préconcentration et l'analyse :
- de substances explosives dans des solutions aqueuses et/ou
- d'UXO et de résidus de munitions en mer et sur les fonds marins
dans laquelle
le dispositif en tant que système (1) comprend au moins un boîtier (3) dans lequel sont prévus au moins un premier module de système de fluide (4) et/ou un deuxième module de système de fluide (30) et au moins un module de système d'analyse (22), comprenant les composants suivants dans le premier module de système de fluide (4):
- au moins trois pompes HPLC (5, 14, 15), de capacités de pompage différentes ;
- au moins deux valves (10, 11) ;
- au moins une valve de sélection d'échantillons (6), sous la forme d'une valve à 6 voies avec des options de réglage commandables pour les positions de rejet et d'injection ;
- au moins un filtre en ligne (13) ;
comprenant les composants suivants dans le deuxième module de système de fluide (30) :
- au moins une pompe HPLC (36) et
- au moins une carte de commande électronique (35)
et comprenant les composants suivants dans le module du système d'analyse (22) :
- au moins un mélangeur (23) ;
- au moins une valve (26), servant de valve de dérivation ;
- au moins un dispositif de chauffage de colonne HPLC (25) ;
- au moins un système UV (24) comprenant un guide d'ondes capillaire et un spectrophotomètre ;
**caractérisé en ce que**
- le premier module de système de fluide (4)
- comporte au moins une pompe à seringue (16) avec une valve de distribution à 4 voies (17) et
- les au moins deux valves (10, 11) disposent de positions de transfert et de chargement/injection commandables ;
- le module de système d'analyse (22) dispose d'au moins un ESI-MS (27) ;
- le deuxième module de système de fluide (30) comporte
- un boîtier sous pression (34) ;
- un premier bouchon d'extrémité A (31) du boîtier sous pression ;
- un deuxième bouchon d'extrémité B (37) du boîtier sous pression ;
- au moins un sélecteur de colonnes HPLC (32), comprenant au moins deux colonnes HPLC (33), servant de colonnes d'extraction en phase solide pour le prélèvement d'échantillons ;
- en présence d'un deuxième module de système de fluide (30), au moins l'un des bouchons d'extrémité du boîtier sous pression (31, 37) comporte des éléments de connexion qui assurent une connexion amovible entre le deuxième module de système de fluide (30) et un module de système d'analyse (22) correspondant.

2. Procédé de prélèvement, d'analyse et de traitement d'échantillons à l'aide d'un dispositif formant système (1) selon la revendication 1, comprenant au moins les étapes suivantes:
a. pré-nettoyage (100)
b. chargement de l'échantillon (101)
c. transfert (102)
d. préparation finale de l'échantillon (103)
e. séparation chromatographique (104)
f. détection par spectrométrie de masse (MS) (105)
g. détection spectrophotométrique dans le domaine ultraviolet (UV) (106)
h. post-purification (107).

3. Procédé de prélèvement, d'analyse et de traitement d'échantillons selon la revendication précédente,
**caractérisé en ce**
**qu'**un échantillon suivant ou un échantillon discret d'un prélèvement est chargé simultanément sur la première colonne d'extraction en phase solide (9) dès qu'un premier échantillon atteint l'étape c. Transfert (102).
